# EUROPEAN PATENT SPECIFICATION

(11) **EP 3 527 120 B1**
(45) Date of publication and mention of the grant of the patent: **23.11.2022**
(21) Application number: 16918808.3
(22) Date of filing: 14.10.2016
(51) Int. Cl.: A61B 3/024, A61B 3/113, G02B 27/01, A61H 5/00

(54) **VIRTUAL REALITY HEADSET FOR VISUAL NEUROREHABILITATION**
VIRTUAL-REALITY-HEADSET FÜR VISUELLE NEUROREHABILITATION
CASQUE DE RÉALITÉ VIRTUELLE POUR LA NEURORÉHABILITATION VISUELLE

(43) Date of publication of application: 21.08.2019
(73) Proprietor: Rapture Games, S.L., 47014 Valladolid (ES)
(72) Inventor: FINAT SAEZ, Jaime, 47150 Simancas (Valladolid) (ES); COCO MARTIN,, Maria Begoña, 47012 Valladolid (ES)
(74) Representative: Pons Ariño, Angel
(86) International application number: PCT/ES2016/070728
(87) International publication number: WO 2018/069555

(56) References cited:
- GB-A- 2 295 685
- GB-A- 2 295 685
- US-A- 6 045 227
- US-A- 6 045 227
- US-A1- 2013 308 099
- US-A1- 2013 308 099
- US-A1- 2014 313 488
- US-A1- 2014 327 880

## Description

### TECHNICAL FIELD OF THE INVENTION

The present invention belongs to the field of stereoscopic vision optical devices, specifically that of virtual reality headsets with eye-tracking, combined in the field of visual neurorehabilitation.

### BACKGROUND OF THE INVENTION

### Campimetry techniques

One of the most significant characteristics of an optical device is the visual field which could be defined as the physical space which a user is capable of perceiving while he fixes his glance on a specific point. There are multiple causes which can alter the visual field of a user, either reducing its amplitude or generating blind spots. Among these causes, the following can be highlighted: glaucoma, brain tumours, trauma of the optical route, brain infarcts, arterial or venous occlusions of the retina, optical neuritis, ischemic optic neuropathy, retinal detachment and diabetic retinopathy.

Visual campimetry is an ophthalmic examination which studies the alterations of the visual field. If there are losses or reductions of the visual field, it is useful to carry out successive campimetries to obtain evolutive control of the same. In this manner, the campimetry is used as a functional test which complements the morphological image tests, such as computerised axial tomography (CAT) or magnetic nuclear resonance.

In general, we can classify the type of campimetries into manual techniques and computerised techniques.

Among the manual techniques are found, the confrontation technique, the tangent screen, Amsler grid and Goldmann perimetry are included. The problem with these techniques is that they are not very accurate and it takes a long time to perform them. They are useful as superficial evaluations in which it is simply desired to observe significant damage, but they do not allow injuries to be detected early since they are not sufficiently refined techniques.

Among the most well-known and used computerised techniques are found Humphrey campimetry and FDT matrix. Said techniques also have some disadvantages or limitations, such as the learning factor, significant variability, the effects of fatigue and the need for greater collaboration by the user.

### Neurorehabilitation

Neurorehabilitation is a process focused on the recovery of the nervous system after a neurological injury, which has the aim of minimising and compensating the functional alterations. Over time different techniques and methodologies have been developed to address the neurorehabilitation process of the affected users.

Specifically, in the field of vision, visual therapy has been a useful tool for addressing the neurorehabilitation of the users by means of performing exercises aimed at improving fixation, tracking, saccadic movements, the stability of the visual image, oculo-manual coordination, the sensitivity to light, recognition and suitable interpretation of the visual image, spatial perception, imagination and construction of the image. In addition, it has been attempted to improve the extension of the visual field of affected users, and make them aware of the same and therefore achieve improved usage of the remaining visual field of each user.

There are some problems associated with the traditional techniques of visual therapy. Firstly, almost all the techniques used thus far are largely dependent on the presence, control and supervision by a professional. This means that the user does not usually have the capacity to carry out the therapies outside of the clinic/hospital environment and if he can perform said activities, the results obtained are not as good. The second problem, derived in part from the above problem, occurs when the users leave the clinic/hospital environment since in many cases the rehabilitation process is interrupted. Thirdly, the specialists affirm that one of the reasons for failure in the treatments is the lack of motivation of the users to perform the indicated exercises. This lack of motivation is due to the proposed exercises usually being repetitive and boring, consequently the user does not carry them out in a suitable manner and in a manner prolonged over time.

At present, attempts are being made aimed at using computer programs (software) in the form of videogames intended as visual rehabilitation techniques which substitute the classic techniques, thus being more attractive and more motivating for the rehabilitation process. The company, Davalor has recently presented an automated visual evaluator (AVE) in which a visual neurorehabilitation program is included using videogames ((blog.davalorsalud.com/, www.bestake.com/es/Proyecto-vision). There are other projects aimed at using videogames as a therapeutic tool intended for visual neurorehabilitation. For example, Kinect SKILLGAMES (skillgames.kinectfordevelopers.com/) is a pack of four computer games which use the Kinect^{®} device as a game controller and which allows that the act of performing the recovery exercises is not such a laborious, repetitive and boring task. However, in this case, the games are specifically intended for motor rehabilitation, not visual rehabilitation, consequently the improvements achieved in vision will be side effects, in any case.

Moreover, the technology of eye tracking is known, the main fields of commercial application of which include assistive technologies for persons with reduced mobility and research in behavioural sciences, with branches in marketing, neuroscience and psychology.

Although less known, there are uses of movement tracking technology which are extending to other areas such as its application to the world of videogames (Isokoski, P., & Martin, B (2006). Eye tracker input in first person shooter games. Proceedings of the 2nd Conference on Communication by Gaze Interaction: Communication by Gaze Interaction-COGAIN 2006: Gazing into the Future, page 78-81). Specifically, it has been demonstrated that it is possible to use this technology to facilitate and even carry out tasks in medical disciplines related to the eye and vision from diagnostic and measuring activities (Langenegger, S. J., Funk, J., & Toteberg-Harms, M. (2011). Reproducibility of retinal nerve fiber layer thickness measurements using the eye tracker and the retest function of Spectralis SDOCT in glaucomatous and health control eyes. Invest Ophthalmol Vis. Sci, 52(6). Pages 3338-3344) to visual rehabilitation therapies (Kasten, E., Bunzenthal, U., & Sabel, B. A. (2006). Visual field recovery after vision restoration therapy (VRT) is independent of eye movements: an eye tracker study. Behavioural brain research, 175(1), pages 18-26).

On the other hand, the latest tendencies in diagnostic and rehabilitation therapies for problems of vision are strongly tending towards the use of virtual reality environments (Rizzo, A., & Kim, G. J. (2005). A SWOT analysis of the field of virtual reality rehabilitation and therapy. Presence, 14(2), pages 119-146; Shiratuddin, M. F., Hajnal, A., Farkas, A., Wong, K. W., & Legradi, G. (2012, June). A proposed framework for an interactive visuotactile 3D virtual environment system for visuomotor rehabilitation of stroke patients. In Computer & Information Science (ICCIS), 2012 International Conference on (Vol. 2., pages 1052-1057, IEEE; Negut, A., Matu S.A., Alin Sava, F., David, D. (2016). Virtual reality measures in neuropsychological assessment: a meta-analytic review. The Clinical Neuropsychologist, 30(2), pages 165-184), including specifically evaluating and rehabilitating users who have suffered partial vision loss.

Due to the characteristics of the current devices, there is a need to incorporate ocular tracking activities in virtual reality environments. These environments are usually implemented with opaque headsets to project the stereoscopic simulation in each one of the eyes of the user, but as they are opaque, they prevent the use of eye-tracking devices. The solution which is being given is to include eye-tracking within the virtual reality headsets (as "eye-tracking HMD upgrade" of SMI-Senso Motoric Instruments for Oculus Rift^{®} headset).

Documents US6,045,227, GB 2295685, and US 2013/308099 relate to headsets for testing a patient that do not comprise infra-red illumination means that are directed to screens such that their light is emitted towards the screens, rebounds on the screens and then hits on the eyes of the patient.

Due to this, the need has been observed to develop products allowing the eye-tracking functionality to be implemented within a virtual reality simulation and which have an affordable price and easy distribution.

### Disadvantages of the prior art

The campimetry techniques used at present and considered as the gold standard use a Humphrey campimetry and have some drawbacks. Firstly, it is necessary for the user to clearly understand the instructions and have good hand-eye coordination which is not present in users with visual field alterations following acquired brain damage. Secondly, in the current measurement techniques it is crucial for the evaluator to be permanently alert to possible movements of the head of the user which would be used as a compensatory strategy for the visual field deficiency and which without doubt introduce errors and alterations in the results of the campimetry tests. Thirdly, the current techniques last a long time which causes fatigue and tiredness in the users and lead to errors during the performance of the test, which can alter the actual results of the evaluated subjects. Lastly, the techniques used at present require the user to be able to remain in a seated position and even for them to be capable of moving to perform it which in many case means they are not able to perform these evaluations in the first moments following some events such as ictus, cranioencephalic trauma, etc., due to many of these users have to remain in bed until their state develops.

At present, some approximations to the use of computer programs have been performed in the form of videogames for their use in the visual rehabilitation process. One of the most advanced projects in this line is that presented by the cited company, Davalor, whose automated vision evaluator includes a visual neurorehabilitation program based on videogames. However, although it is a good approximation, this device has clear disadvantages. Firstly, it is a device only accessible for clinics/companies due to its relatively high cost. This means that it does not eliminate the problem associated with the need for a user to have to move to the clinic or optician to perform his visual neurorehabilitation exercises. In addition, this device is not portable, therefore there are multiple situations in which it cannot be used. But more important is the fact that the users should be able to remain in the seated position during the performance of the test which means that it is not applicable in early stages of the neurorehabilitation process, such as for example in the first days after a cerebrovascular accident.

On the other hand, due to the fact that they do not reach to the general public, the existing solutions, which include eye-tracking within a virtual reality headset, are relatively expensive, difficult to find, less integrated and highly specialised: the hardware is available commercially, but it is not a complete solution aimed at the patient; in order to implement a solution of this type it will be necessary to find IT specialists, such as specialised personnel who install the devices as part of a complete system and even develop the specific diagnostic and rehabilitation applications.

### DESCRIPTION OF THE INVENTION

The present invention is defined by the independent claim,
while the dependent claims describe other characteristics of the same. Also described herein is a kit, not forming part of the invention, for adapting a virtual reality headset and its method, not forming part of the invention,
of use for visual neurorehabilitation, both diagnosis and rehabilitation, which is simple and economic, consequently accessible to the general public. The technical problem to be resolved is to configure the kit and establish the steps for using the same to arrive at the cited object.

The virtual reality headset comprises a case with an opening adaptable to the face of a user, in the sense that the hollow which is the opening follows the contour of the face of a user, therefore, inherently the material which surrounds the opening has a flexibility which allows this, such as is known in the virtual reality headsets of the prior art; in the case, there are two screens connected to corresponding lenses facing the eyes of the user, as is known, the screens are arranged aligned to one another and each one of them is facing a lens and it is facing an eye.

The virtual reality headset is characterised in that it comprises a mask as a central structure or support, normally made of plastic material, although it could be made of another material, preferably lightweight so as not to put weight on the head of the user which is arranged inside the case, that is to say, it is introduced as an internal component of the same, the mask comprises two holes facing the screens, as it is inherently understood, one hole faces each screen, the lenses are fixed to the mask corresponding to the holes, two cameras, in the same sense as has been cited, one camera for each eye, are fixed to the mask in proximity to the holes by means of fastening means such that they are directed towards the user, that is to say, they are fixed such that their objective points to the eye of the user, normally by way of the hole; infra-red illumination means, one for each eye, are fixed to the mask such that their beam is directed towards the user, the infra-red illumination means and the camera are connected to a control device which, in turn, comprises data storage media and a processor.

The infra-red illumination means emit infra-red light within a safe range, with an illumination intensity always below the emissivity of the white of the screens, normally an OLED type screen. In addition, the infra-red light does not directly hit on the eyes of the user since this light does not causes aberration effects and does not produce an irritating sensation so that the eye blinks with greater frequency; as such, if an excessively high emission intensity is used, damage could be caused. In order to avoid this, the opportune measurement of the intensity of the signal have been performed and when various illumination means are placed the distance therebetween is maximised since by being very close the intensity can enter in phase and build up, and consequently the intensity could be duplicated in some regions. According to the invention, the illumination means are arranged such that their light is emitted towards the screens, rebounds and then hits on the eyes of the user since the intensity is inversely proportional to the square of its distance; consequently, with its distance increased, the intensity of the signal is reduced in a quadratic manner.

Said data storage media include a user eye-tracking program and a campimetry program. These programs by themselves and separately are known in the prior art. They are required in combination with the rest of the mentioned characteristics to achieve the additional technical effect corresponding to the object of the invention for achieving visual neurorehabilitation of the user. It is understood that the functioning of the programs is normal and an extensive explanation is not necessary as they are orders which cause the processor to send the signals and pertaining orders to the devices to which it is connected, normally by means of interfaces, elements, electronic modules of those known for their functioning.

Similarly, disclosed herein is a method, not forming part of the invention, for using a kit for adapting a virtual reality headset for the visual neurorehabilitation of a user, the kit being as cited here above.

The method not forming part of the invention is characterised in that it comprises the following steps for diagnosing losses in visual field:
- suprathreshold stimulation such that it guarantees a contrast visible by the user, preferably it relates to that corresponding to the suprathreshold modality of the SITA Fast test;
- glance of the user at a particular area determined within the visual field in the headset;
- storage of the position of the centre of the pupil in the data storage media by means of a user eye-tracking program;
- obtaining a map of the visual field of the user in black and white by means of a campimetry program, the white corresponding to an area where the user has suprathreshold visibility, the black to a blind spot for the user.

An advantage of the invention is an improved measurement of the fixation losses. In cases such as those which the user has an alteration of the visual field such as hemianopsia or quadrantanopia of some type, his blind spot cannot be precisely determined, since the disorder of the vision itself prevents correct estimation of the same. The Humphrey campimetry uses statistical data relating to the biological and environmental factors of the user. The invention follows the very centre of the pupil at the time when the user is looking at the point of interests. From this moment, at intervals of milliseconds, the movements of the eye and the variation of its position are measured; as such, greater accuracy is obtained, measured more often and applying the same discard scale (20% of losses of fixation detected).

Another advantage is that the kit, not forming part of the invention, is a low-cost device. While a Humphrey campimetry is a complex device with a relatively high cost, the kit not forming part of the invention is much more economic, in the order of 10 times less.

Another advantage is that the diagnosis and rehabilitation is performed in the same device, herein understood as the kit with the headset. In addition to the advantages previously mentioned, the same device can serve not only as a diagnostic tool for the preferred SITA 24-2 campimetry, but also allows oculomotor exercises to be performed by tracking moving figures which are directed towards the blind spots of the user which is a program that takes the form of a videogame (to increase the motivation of the patient), in the same environment and using the accurate data generated during the diagnostic session.

Another advantage is that the rehabilitation is effective by being entertaining. In accordance with the above advantage, as videogames are elements with a significant leisure component, they allow learning and training, in a natural and entertaining manner, of abilities in the human brain, such as reflexes, memory, decision making, hand-eye coordination, identification of patterns, tracking moving objects, speed of the user to converge and accommodate points in 3D space, etc. The user, as he is immersed and concentrating on the tasks which require his visual capacity, trains his abilities; as he is doing it within a playful environment, it results in less costly effort and more time the user can be concentrated thanks to the "prizes" and the "rewards" which he obtains within the environment. If the novelty of the virtual reality is added to this, and the immersive capacity of the device (which makes the user to feel inside the environment), the rehabilitation is successful, effective and the most efficient possible, in terms of time.

Another advantage is that it is a portable device, herein understood as the kit with the headset. Unlike other devices on the market which incorporate diagnosis and/or rehabilitation such as the Humphrey campimetry or the Davalor device, the system proposed is portable and can be moved to the very bed of a hospital for early intervention. At the same time, it can be incorporated into any optometric or ophthalmological clinic, as it does not require any special installation.

Another advantage is that the device, herein understood as the kit with headset, is comfortable and ergonomic. In addition to being able to move the invention comfortably with any type of cart with wheels, the device in itself is comfortable to use as it is, as a whole, a virtual reality headset like those known.

Another advantage is the possibility to modify the sizes of the stimulations inside the visual field between 0.043° and 0.86°. The Humphrey campimetry allows the optometrist to select 4 possible stimulation sizes (I, II, III and IV). The invention, when generating the stimulation within a virtual device, herein understood as the kit with the headset, has the capacity to modify the scale of the stimulation and its contrast with the background in a much more precise manner, within the scale of real numbers with up to 5 decimal places of precision, which means, for practical purposes, a continuous scale.

Another advantage is that it is not necessary for the user to be in a predetermined position, such as seated. Traditionally the head of the user is fixed to a type of chinrest device since, instinctively and naturally, a user with a condition of the visual field, tends to perform movements with his head to compensate the parts of the field which he does not perceive, either due to an oculomotor problem or due to a condition of the visual system at some point. The use of the virtual reality headset by itself eliminates this problem since, as it is fixed to the face of the user, the body movements and head movements do not affect the virtual environment that he is observing. In the case of immersive virtual reality videogames in which the movement of the head effectively modifies the part of the virtual environment that the user is watching, normally virtual reality headsets include a series of accelerometers, gyroscopes or systems for interpreting the external orientation based on the analysis and tracking of a series of positional markers on the headset; with these elements, it is known precisely, with a delay of milliseconds, the exact orientation of the head such that the entire system readjusts to this orientation. Therefore, if the user moves his head to try to perceive a specific area or a stimulation, it is not going to be useful, since the videogame is designed so that the stimulations move in accordance with the orientation of the device, herein understood as the kit with headset. If the user does not perceive the stimulation with a determined orientation, he will not perceive it with another one. This means that the system is more reliable and more comfortable for the user (has no reason to be firmly fixed to a device, but rather can explore with comfort, moving freely), and for the professionals who supervise (who now do not have to be as alert to possible movements of the head of the user).

### BRIEF DESCRIPTION OF THE FIGURES

The present specification is complemented by a set of figures, illustrative of the preferred example and not limiting of the invention.
Figure 1 represents an exploded perspective view of the mask of the kit in relation to the case and the lenses of the headset; the control device is represented schematically.
Figure 2 represents a perspective view of means for regulating the position of a camera.
Figure 3 represents the step of the user viewing a particular area determined within the visual field of the headset, the field represented as the screens of the headset.
Figure 4 represents the step of storing the position of the centre of the pupil in the data storage media and the confidence region around the pupil.

### DETAILED DESCRIPTION OF THE INVENTION

An embodiment of the invention is outlined below with the aid of the figures.

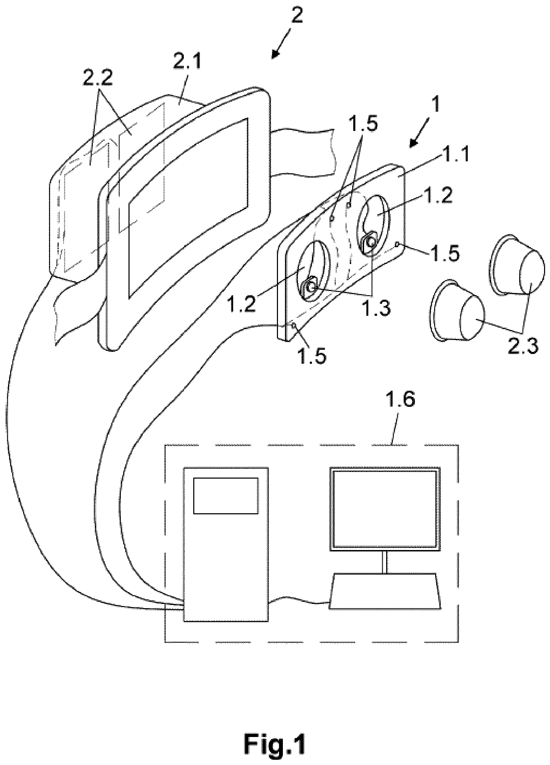

In Figure 1 a kit, not forming part of the invention, for adapting (1) a virtual reality headset (2) for the visual neurorehabilitation of a user is shown, said headset comprises a case (2.1) with an opening adaptable to the face of the user, in the case (2.1) two screens (2.2) are arranged connected to corresponding lenses (2.3) facing the eyes of the user. The kit (1) comprises a mask (1.1) which is arranged inside the case (2.1), the mask (1.1) comprises two holes (1.2) facing the screens (2.2), the lenses (2.3) are fixed to the mask (1.1) corresponding to the holes (1.2), two cameras (1.3) are fixed to the mask (1.1) in proximity to the holes (1.2) by means of fastening means (1.4) such that they are directed towards the user, said fastening means (1.4) not indicated in Figure 1 can be in their simplest form, an adhesive, a weld or even a clip.

In Figure 1 infra-red illumination means (1.5) are shown which are fixed to the mask (1.1) such that their beam is directed towards the user, specifically two LEDs arranged diagonally on both sides of each hole (1.2) to guarantee optimal illumination, although using one LED on the entire mask (1.1) could be sufficient. The cameras (1.3) and the screens (2.2) and the infra-red illumination means (1.5) are connected to a control device (1.6). The connection is represented by means of conductor wires in the manner of a schematic representation, its specific configuration will depend on the desired embodiment, wire conductors possibly being combined in the same wiring harness, implemented by wireless communication when possible, etc.

The control device (1.6), in turn, comprises data storage media such as a hard disk or flash memory or any other type and a processor, any of those known, to thus form a data processing device or computer of those known such as a desktop PC, the one represented, or a laptop, a tablet etc. The data storage media include a user eye-tracking program and a campimetry program.

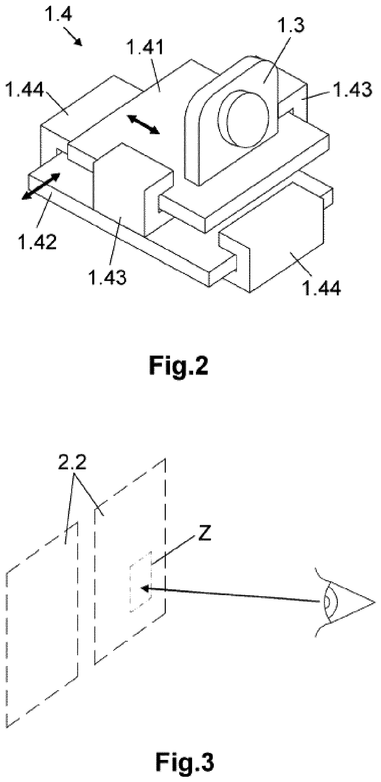

Optionally, the fastening means (1.4) of the cameras (1.3) to the mask (1.1) comprise means for regulating the position; specifically, as represented in Figure 2, said position regulation means comprise a first platform (1.41) movable in a first direction arranged on a second platform (1.42) in turn arranged perpendicularly to the first (1.41); in the embodiment shown, the second platform (1.42) has fixed a first clamp (1.43) within which the first platform moves (1.41) linearly; analogically, the mask (1.1), not represented in Figure 2, has fixed a second clamp (1.44) within which the second platform (1.42) moves linearly, with this simple configuration, orthogonal displacement is achieved between both platforms. Although not represented, the positional adjustment of each camera (1.3) can be implemented in a motorised manner according to the outlined embodiment or any other suitable for positional adjustment.

Optionally, the data storage media include a moving figure tracking program, such as for example in the manner of a videogame.

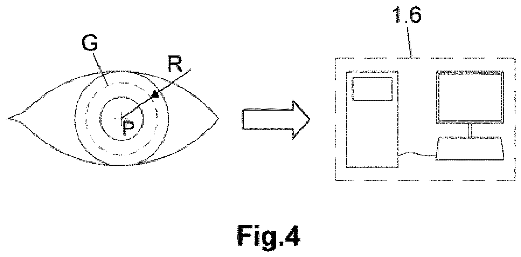

The method, not forming part of the invention, for using a kit for adapting (1) a virtual reality headset (2) for visual neurorehabilitation of a user according to the kit (1) described comprises the following steps for the diagnosis of losses in visual field:
- suprathreshold stimulation such that it guarantees a contrast visible by the user;
- glance of the user at one particular area (Z) determined within the visual field in the headset, as is represented in Figure 3, the visual field is considered, for the sake of the simplicity of the figures, one of the screens (2.2), although it could vary in size and position with respect to these;
- storage of the position (P) of the centre of the pupil in the data storage media by means of a user eye-tracking program, as is represented in Figure 4;
- obtaining a map of the visual field of the user in black and white by means of a campimetry program, the white corresponding to an area where the user has suprathreshold visibility, the black to a blind spot for the user.

Optionally, the diagnosis is accepted or discarded based on the following steps:
- determining a confidence region (G) of predetermined radius (R) around the pupil, as is represented in Figure 4;
- if the centre of the pupil is displaced outside of the confidence region (G), a time counter is initiated which is stopped upon return to the confidence area;
- if, upon completing the test, the time counter outside of the confidence region (G) represents more than 20% of the total time of the test, it is understood that the user has not maintained the fixation and the test is considered invalid, otherwise, it is considered valid.

This manner of accepting or discarding the test is an alternative to what is known, like in the SITA Fast 24-2 test which forms the preferred standard and is implemented in the Humphrey campimetry. A panel of lights has been created inside the virtual world which are illuminated at random, in the same manner as in the Humphrey test. The user also carries a remote with a button to define when he has seen a stimulation and when he has not perceived it to reduce the false positives (keystroke of the user without visual stimulation shown), there is an audio effect which is reproduced at regular intervals, whether there is visual stimulation or not, thus it can be determined if the user presses due to the sound to falsify the results and therefore it is necessary to repeat the test.

Optionally, the size of the stimulation within the visual field can be configured to vary between 0.043° and 0.86°.

Another option is that after the diagnosis, rehabilitation is carried out by means of tracking moving figures which are directed towards the blind spots of the user, that is to say, in the form of a videogame, with the advantages cited in the description section of the invention. Said videogame for example shows spaceships which has as objective to make the user look at a determined position to be able to recover part of the sensitivity or the field amplitude of the user after loss in visual field due to an injury due to brain infarct; the videogame uses the interpretation of the direction of the glance of the user as a shooting "pointer" and measures the time to fix the glance on a determined moving object, if this fixation time exceeds a limit, normally pre-established by a physician, the element is eliminated from the screen and it is considered that the user fixed his glance for sufficient time on the object.

## Claims

1. A virtual reality headset (2) for the visual neurorehabilitation of a user, comprising:
a case (2.1) with an opening adaptable to the face of the user, in the case (2.1) two screens (2.2) are arranged aligned to one another and each one of them facing corresponding lenses (2.3) facing the eyes of the user, and a mask (1.1) which is arranged inside the case (2.1), the mask (1.1) comprises two holes (1.2) facing the screens (2.2), the lenses (2.3) are fixed to the mask (1.1) corresponding to the holes (1.2), two cameras (1.3) are fixed to the mask (1.1) in proximity to the holes (1.2) by means of fastening means (1.4) such that they are directed towards the user, infra-red illumination means (1.5) are fixed to the mask (1.1) and are directed to the screens (2.2), such that their light is emitted towards the screens, rebounds on the screens (2.2) and then hits on the eyes, the infra-red illumination means (1.5) and the cameras (1.3) are connected to a control device (1.6) which, in turn, comprises data storage media and a processor, the data storage media include a user eye-tracking program and a campimetry program.

2. The virtual reality headset (2) according to claim 1, wherein the fastening means (1.4) of the cameras (1.3) to the mask (1.1) comprise position regulation means, which comprises a first platform (1.41) movable in a first direction and arranged on a second platform (1.42), movable in a second direction perpendicular to the first direction.

3. The virtual reality headset (2) according to claim 1, wherein the infra-red illumination means (1.5) are at least one LED.

4. The virtual reality headset (2) according to any of the preceding claims, wherein the data storage media include a moving figure tracking program.

5. The virtual reality headset (2) according to any of the preceding claims, wherein the fastening means (1.4) are motorised.

6. The virtual reality headset (2) according to any of the preceding claims, wherein the infra-red illumination means (1.5) are placed at a maximum distance therebetween.

7. The virtual reality headset (2) according to any of the preceding claims, further comprising a series of accelerometers, gyroscopes or systems for interpreting the external orientation based on the analysis and tracking of a series of positional markers on the headset; the headset (2) further configured to determine the exact orientation of the head of a user such that stimulations move in accordance with the orientation of the headset.

## Patentansprüche

1. Virtual-Reality-Headset (2) für die visuelle Neurorehabilitation eines Benutzers, umfassend:
ein Gehäuse (2.1) mit einer an dem Gesicht des Benutzers anpassbaren Öffnung, wobei in dem Gehäuse (2.1) zwei Bildschirme (2.2) zueinander fluchtend angeordnet sind und jeder von ihnen entsprechenden Linsen (2.3) zugewandt ist, die den Augen des Benutzers zugewandt sind, und eine Maske (1.1), die im Inneren des Gehäuses (2.1) angeordnet ist, wobei die Maske (1.1) zwei Löcher (1.2) umfasst, die den Bildschirmen (2.2) zugewandt sind, wobei die Linsen (2.3) an der Maske (1.1) entsprechend den Löchern (1.2) befestigt sind, zwei Kameras (1.3) an der Maske (1.1) in der Nähe der Löcher (1.2) mit Hilfe von Befestigungsmitteln (1.4) so befestigt, dass sie auf den Benutzer gerichtet sind, wobei Infrarot-Beleuchtungsmittel (1.5) an der Maske (1.1) befestigt und auf die Bildschirme (2.2) gerichtet sind, so dass ihr Licht in Richtung der Bildschirme emittiert wird, auf den Bildschirmen (2.2) prallt und dann auf die Augen trifft, wobei die Infrarot-Beleuchtungsmittel (1.5) und die Kameras (1.3) sind mit einer Steuervorrichtung (1.6) verbunden sind, die ihrerseits Datenträger und einen Prozessor umfasst, wobei die Datenträger ein Benutzer-Augenverfolgungsprogramm und ein Kampimetrieprogramm enthalten.

2. Virtual-Reality-Headset (2) nach Anspruch 1, wobei die Befestigungsmittel (1.4) der Kameras (1.3) an der Maske (1.1) Positionsregulierungsmittel umfassen, die eine erste Plattform (1.41) umfassen, die in einer ersten Richtung bewegbar ist und auf einer zweiten Plattform (1.42) bewegbar in einer zweiten Richtung senkrecht zur ersten Richtung angeordnet ist.

3. Virtual-Reality-Headset (2) nach Anspruch 1, wobei die Infrarot-Beleuchtungsmittel (1.5) mindestens eine LED sind.

4. Virtual-Reality-Headset (2) nach einem der vorhergehenden Ansprüche, wobei die Datenträger ein Bewegungsfigur-Verfolgungsprogramm enthalten.

5. Virtual-Reality-Headset (2) nach einem der vorhergehenden Ansprüche, wobei die Befestigungsmittel (1.4) motorisiert sind.

6. Virtual-Reality-Headset (2) nach einem der vorhergehenden Ansprüche, wobei die Infrarot-Beleuchtungsmittel (1.5) in einem maximalen Abstand dazwischen angeordnet sind.

7. Virtual-Reality-Headset (2) nach einem der vorangehenden Ansprüche, ferner umfassend eine Reihe von Beschleunigungsmessern, Gyroskopen oder Systemen zum Interpretieren der externen Orientierung basierend auf der Analyse und Verfolgung einer Reihe von Positionsmarkierungen auf dem Headset; wobei das Headset (2) ferner so konfiguriert ist, dass es die exakte Ausrichtung des Kopfes eines Benutzers bestimmt, so dass sich Stimulationen gemäß der Ausrichtung des Headsets bewegen.

## Revendications

1. Casque de réalité virtuelle (2) pour la neuroréhabilitation visuelle d'un utilisateur, comprenant :
un boîtier (2.1) avec une ouverture adaptable au visage de l'utilisateur, dans le boîtier (2.1) deux écrans (2.2) sont agencés alignés l'un à l'autre et chacun d'eux faisant face à des lentilles correspondantes (2.3) faisant face aux yeux de l'utilisateur, et un masque (1.1) qui est agencé à l'intérieur du boîtier (2.1), le masque (1.1) comprend deux trous (1.2) faisant face aux écrans (2.2), les lentilles (2.3) sont fixées au masque (1.1) correspondant aux trous (1.2), deux caméras (1.3) sont fixées au masque (1.1) à proximité des trous (1.2) au moyen de moyens d'attache (1.4) de sorte qu'elles sont dirigées en direction de l'utilisateur, des moyens d'éclairage infrarouge (1.5) sont fixés au masque (1.1) et sont dirigés vers les écrans (2.2), de sorte que leur lumière est émise en direction des écrans, rebondit sur les écrans (2.2) puis arrive sur les yeux, les moyens d'éclairage infrarouge (1.5) et les caméras (1.3) sont connectés à un dispositif de commande (1.6) qui, à son tour, comprend des supports de stockage de données et un processeur, les supports de stockage de données incluent un programme de suivi des yeux de l'utilisateur et un programme de campimétrie.

2. Casque de réalité virtuelle (2) selon la revendication 1, dans lequel les moyens d'attache (1.4) des caméras (1.3) au masque (1.1) comprennent des moyens de réglage de position, qui comprennent une première plateforme (1.41) pouvant se déplacer dans une première direction et agencée sur une seconde plateforme (1.42), pouvant se déplacer dans une seconde direction perpendiculaire à la première direction.

3. Casque de réalité virtuelle (2) selon la revendication 1, dans lequel les moyens d'éclairage infrarouge (1.5) sont au moins une DEL.

4. Casque de réalité virtuelle (2) selon l'une quelconque des revendications précédentes, dans lequel les supports de stockage de données incluent un programme de suivi de personnage se déplaçant.

5. Casque de réalité virtuelle (2) selon l'une quelconque des revendications précédentes, dans lequel les moyens d'attache (1.4) sont motorisés.

6. Casque de réalité virtuelle (2) selon l'une quelconque des revendications précédentes, dans lequel les moyens d'éclairage infrarouge (1.5) sont placés à une distance maximale entre eux.

7. Casque de réalité virtuelle (2) selon l'une quelconque des revendications précédentes, comprenant en outre une série d'accéléromètres, de gyroscopes ou de systèmes pour interpréter l'orientation externe en fonction de l'analyse et du suivi d'une série de marqueurs de position sur le casque ; le casque (2) étant en outre configuré pour déterminer l'orientation exacte de la tête d'un utilisateur de sorte que les stimulations se déplacent conformément à l'orientation du casque.
